# EUROPÄISCHE PATENTSCHRIFT

(11) **EP 0 371 265 B1**
(45) Veröffentlichungstag und Bekanntmachung des Hinweises auf die Patenterteilung: **12.01.1994**
(21) Anmeldenummer: 89119976.2
(22) Anmeldetag: 27.10.1989
(51) Int. Cl.: G01N 33/543, G01N 33/553

(54) **Verfahren und Vorrichtung zur Messung der Chemilumineszenz**
Method and apparatus for measuring chemiluminescence
Mesure de chimioluminescence

(30) Priorität: 01.12.1988 DE 3840462
(43) Veröffentlichungstag der Anmeldung: 06.06.1990
(73) Patentinhaber: LABORATORIUM PROF. DR. RUDOLF BERTHOLD GmbH & Co. KG, 75323 Bad Wildbad (DE)
(72) Erfinder: Berthold, Fritz, Dr., D-7530 Pforzheim (DE); Lohr, Willy, D-7547 Wildbad (DE)
(74) Vertreter: Frank, Gerhard, Dipl.-Phys.

(56) Entgegenhaltungen:
- EP-A- 0 149 565
- EP-A- 0 262 760
- EP-A- 0 265 244
- EP-A- 0 270 206
- DE-A- 3 623 601
- DE-C- 1 498 857

## Beschreibung

Die Erfindung betrifft ein Verfahren zur Messung der Chemilumines - zenz mittels eines Photomultipliers, beispielsweise bei Immunoassays oder an Nukleinsäurehybriden unter Verwendung von magnetischen Mikropartikeln.

Chemilumineszenzverfahren beruhen auf der Lichtemission von bestimmten Molekülen infolger einer chemischen Reaktion, bei der diese Moleküle in energetisch angeregte Zustände versetzt werden, von wo aus sie unter Lichtemission in ihren Grundzustand zurückkehren. Derartige Chemilumineszenz- Verfahren sind insbesondere von Bedeutung für den Nachweis von Antigenen (Bakterien), die beispielsweise als Krankheitserreger sich im menschlichen Zellgewebe befinden und eine spezifische Infektion auslösen. Seit einigen Jahren besteht die Möglichkeit, derartige Antigene bzw. Antikörper mit Hilfe der Chemilumineszenz mit der Technik des immuno-assays in kurzer Zeit zu identifizieren. Die hierbei durchgeführten Verfahrensschritte können auch den Einsatz von magnetischen Partikeln einschließen, die eine Hilfsfunktion bei diesen Verfahrensschritten haben.

Bei der EP-0 149 565 werden die magnetischen Micropartikel mit dem Marker wieder in einer Flüssigkeit suspendiert und danach wird unmittelbar die Lumineszenz-Reaktion ausgelöst, also in einem Zustand, bei dem das mit dem Marker versehene Reagenz noch an die Micropartikel gekoppelt ist. Es versteht sich von selbst, daß die in dieser EP-0 149 565 (insbesondere Seite 3 oben) als überraschend dargestellte Erfindung nur dann Anwendung finden kann, wenn die Konzentration der magnetischen Micropartikel in der Lösung einen gewissen Wert nicht überschreitet, da selbstverständlich diese eine Schwächung des Lumineszenzsignals und damit eine Beeinflussung des Meßergebnisses bewirken.

Bei der EP-0 030 086 wird ein Rack beschrieben, in dessen Boden verschiedene Magnete 15 angeordnet sind, auch hier dient diese Anordnung dazu, überschüssige Flüssigkeit aus den Probenröhrchen zu dekantieren, die an die Micropartikel gebundene nachzuweisende Substanz festzuhalten; danach werden die mit dem Marker versehenen magnetischen Partikel ausgemessen (Seite 9 unten), nähere Einzelheiten zu Art und Weise der Messung sind hier nicht aufgeführt.

Bei der WO 86/04684 A1 werden nach entsprechenden Vorbereitungsschritten die magnetischen Partikel mit Hilfe des Magneten in eine besondere Schicht ("separation layer 2") gezogen, so daß hierdurch eine Trennung der gebundenen Marker-Substanz von der freien Marker-Substanz erfolgt und dadurch entweder erstere oder letztere ausgemessen werden kann. Diese Lösung bietet den Vorteil, daß das Dekantieren und Neuansetzen einer Lösung vermieden wird und die Lumineszenz-Messung in der Tat im "ursprünglichen" Probengefäß durchgeführt werden kann. Dieses Verfahren ist jedoch ohne weiteres nur zur Durchführung von Fluoreszenzmessungen geegnet und der zweischichtige Aufbau der Meßlösung, der letztlich das Dekantieren ersetzt, erfordert zusätzlichen Aufwand. Lumineszenzmessungen, bei denen durch Zugabe einer geeigneten Substanz die Lumineszenz-Reaktion erst aktiviert werden muß, sind hiermit nicht ohne weiteres durchführbar, da die Mengenverteilung der hier zuzugebenden, die Lumineszenz initiierenden Substanz auf die beiden Meßschichten zwangsläufig undefiniert ist und folglich die meßtechnische Trennung der resultierenden Lumineszenzreaktion in Signale aus der oberen Schicht und Signale aus der unteren Schicht kaum möglich sein dürfte.

Dieser Vorschlag aus der WO 86/04684 ist daher für die Durchführung von Lumineszenzverfahren ungeeignet.

Ein gattungsgemäßes, speziell weiterentwickeltes Verfahren wurde von der Firma Gen-Probe-Inc. in San Diego, USA, unter der Bezeichnung "Pace Assay System" entwickelt. Es bezieht sich anstelle auf der bei den oben erläuterten Immuno-Assays üblichen Antigen-Antikörper-Komplexbildung auf die Verwendung von ribosomaler RNS und einer hierzu "passenden" einsträngigen DNS.

Ausgangspunkt für diese Überlegungen ist die für den jeweiligen nachzuweisenden Mikroorganismus spezifische ribosomale RNS, die in jeder spezifischen Bakterienzelle in der Größenordnung von etwa 10.000 Kopien vorliegt (rRNS). Diese RNS wird aus Gewebeproben gewonnen. Sie dient als "Target"für eine "genetische Sonde", die spezifisch für die nachzuweisende rRNS ausgewählt wird und aus einer einsträngigen DNS besteht, an die eine chemische Substanz angekoppelt ("labeled") ist, an der dann an der Meßstelle die Chemilumineszenz initiiert wird. Je nach Häufigkeit des Vorkommens der rRNS kann sich eine entsprechende Anzahl von rRNS-DNS-Paaren bilden und somit eine entsprechende Anzahl von Molekülen der Chemilumineszenz-erzeugenden chemischen Substanz. Beim beschriebenen Verfahren wird als Marker hierfür Akridinium-Ester verwendet, es handelt sich bei diesem Beispiel also um eine nicht-radioaktive Nachweismethode.

Treffen somit in einer geeigneten Lösung eine entsprechende Anzahl von rRNS-Molekülketten ("Targets") auf entsprechend spezifische DNS-Sonden , so erfolgt eine spezifische Ankoppelung und damit eine Markierung dieser rRNS-Mokekülketten an die DNS-Sonden, die als Hybridisierung bezeichnet wird, es bilden sich also stabile rRNS-DNS-Hybride, deren Anzahl durch die Anzahl der beim zu testenden Mikroorganismus vorhandenen rRNS-Molekülketten bestimmt ist, da die DNS-Sonden im Überschuß vorhanden sind.

Um diejenigen DNS-Sonden-Molekülketten, die keinen "rRNS-Partner" gefunden haben, und die zu einer Verfälschung des Meßergebnisses führen würden, wieder zu separieren, werden auch bei dem beschriebenen Verfahren der Firma Gen-Probe Inc. paramagnetische Mikropartikel mit einer Hydroxyapatit-Oberflächenbeschichtung eingesetzt, die in der Lösung eine sehr hohe Oberfläche aufweisen und an denen sich die rRNS-DNS-Hybride festsetzen. Durch Einwirkung eines Magnetfeldes werden die derart besetzten paramagnetischen Mikropartikel ortsfest gehalten, so daß die überschüssigen, nicht-hybridisierten DNS-Sonden getrennt werden können. Nach diesem Trennvorgang werden die rRNS-DNS-Hybride wieder von den paramagnetischen Mikropartikeln getrennt, um die Quantenausbeute bei der nachfolgend vorgenommenen Chemilumineszenz-Reaktion und die optische Effizienz bei der anschließenden Messung im Fotomultiplier zu erhöhen.

Unmittelbar vor der Meßstelle wird die die Chemilumineszenz-Reaktion aktivierende Substanz, hier beispielsweise eine alkalische Wasserstoffperoxyd-Lösung injiziert, worauf das an der DNS-Sonde angekoppelte Label (Akridinium Ester) zerfällt und einen Lichtimpuls in der Größenordnung von etwa zwei Sekunden abgibt, dessen Intensität vom Fotomultiplier an der Meßstelle registriert wird. Durch Vergleich der gemessenen Intensität mit vorher gewonnen Standardwerten läßt sich das Vorhandensein des spezifischen Mikroorganismus nachweisen.

Für spezifische Anwendungsfälle ist es wünschenswert, mit einer relativ hohen Konzentration der paramagnetischen Mikropartikel zu messen, die auch während der Injizierung der die Chemilumineszenz aktivierenden Substanz und der nachfolgenden Messung homogen in der Meßlösung verteilt sind. Eine hohe Konzentration dieser paramagnetischen Mikropartikel kann jedoch durch Streuungs- oder Absorbtionsvorgänge die Intensität des Chemilumineszenz-Signals reduzieren mit der Folge, daß unter Umständen das Nachweisergebnis, das ja letztlich durch die Intensität des Lichtimpulses representiert wird, auch von der jeweiligen Konzentration der paramagnetischen Mikropartikel in der Meßlösung abhinge, mit der Folge, daß eine Nicht-Berücksichtigung dieser Konzentration zu einer Verfälschung des Nachweisergebnisses führen könnte.

Es hat daher nicht an Versuchen gefehlt, solche unerwünschten Einflüsse der Magnetpartikel auszuschließen, indem man entweder mit Hilfe von Stangenmagneten die Magnetpartikel aus den Probengefäßen "absaugt" oder indem man (ebenfalls durch Einwirkung eines Magneten) die Magnetpartikel an einer Stelle des Probengefäßes fixiert und dann die Probenflüssigkeit mit dem Analyten entnimmt und umfüllt.

Beide Konzeptionen sind umständlich und aufwendig und fügen den bei Immunoassays ohnehin notwendigen Verfahrensschritten noch weitere hinzu, mit allen Problemen hinsichtlich Arbeitsaufwand, Kosten und Risiken (Vertauschungsgefahr), die mit zunehmender Anzahl von Verfahrensschritten ansteigen.

Aufgabe der Erfindung ist daher eine Vereinfachung beim Ablauf eines solchen Immunoassays durch Einsparung dieser Verfahrensschritte zu erzielen.

Erfindungsgemäß wird diese Aufgabe durch den kennzeichnenden Teil des Patentanspruchs 1 gelöst.

Der Erfindungsgedanke besteht dabei darin, durch ein externes Magnetfeld die paramagnetischen Mikropartikel frühzeitig von der Meßöffnung (Meßfenster) des Fotomultipliers an der Meßstelle "wegzuziehen", so daß die durch die Chemilumineszenz erzeugten Photonen auf ihrem Weg zum Fotomultiplier von diesen paramagnetischen Mikropartikeln nicht mehr behindert werden können, wobei die Magnetpartikel in der Lösung, d.h. im Probengefäß verbleiben können.
Zur Durchführung dieses Verfahrens geht die Erfindung von einer Anordnung aus, bei der die Probengefäße mittels eines gegenüber der Meßstelle angeordneten Sternrades an der Meßstelle vorbeigeführt werden, wo auch die Zugabe der die Chemilumineszenz initiierenden chemischen Substanz erfolgt; eine solche Anordnung ist in der DE-OS 36 23 601 beschrieben und braucht daher im einzelnen nicht erläutert zu werden.

Um die erwünschte Einwirkung des erfindungsgemäß vorgesehenen Magnetfeldes konstuktiv zu verwirklichen, sieht die Erfindung eine Ausgestaltung des Sternrades gemäß dem kennzeichnenden Teil des Patentanspruchs 2 vor: Dadurch, daß die Probengefäße während ihres Kontaktes mit dem Sternrad beim Vorbeitransport an der Meßstelle in fester räumlicher Beziehung mit diesem verbleiben, ist auch die Einwirkung des Magnetfeldes des auf das jeweilige Probengefäß einwirkenden Permanentmagneten konstant und so gerichtet, daß die paramagnetischen Mikropartikel auf die zur Achse des Sternrades gerichtete Innenwandung des Probengefäßes gezogen werden, wo sie sich absetzen, die übrigen Volumenbereiche der Meßflüssigkeit bleiben demnach frei von diesen Mikropartikeln.

Diese Lösung ist konstruktiv besonders einfach, da die vorbekannte Vorrichtung lediglich durch den Einsatz geeigneter Permanentmagneten in diesem Sternrad ergänzt werden muß.

Eine weitere Realisierung des Erfindungsgedankens geht von einer Anordnung aus, bei der jeweils mehrere Probengefäße in einer linearen Anordnung an der Meßstelle vorbeigeführt werden, wie sie beispielsweise in der DE-1 498 857 beschrieben ist, wo eine Gruppe von Probengefäßen in einem starren, gemeinsamen Probengefäßhalter an der Meßstelle vorbeigeschoben oder vorbeigezogen werden.

Hier wird die Umsetzung des erfindungsgemäßen Verfahrens gemäß dem kennzeichnenden Teil des Patentanspruchs 3 erreicht.

Je nach Konzentration der verwendeten paramagnetischen Mikropartikel kann ein unter Umständen relativ starkes Magnetfeld erforderlich sein. Da dieses Magnetfeld sich bis zur Meßstelle, wo in der Regel der Photomultiplier angeordnet ist, erstreckt, kann bei manchen Einsätzen nicht ausgeschlossen werden, daß die Funktionsweise des Photomultipliers durch magnetishe Streufelder beeinträchtigt wird.

Weitere Ausgestaltungen der erfindungsgemäßen Anordnung gemäß den Ansprüchen 4 und 5 stellen sicher, daß derartige Störeffekte ausgeschlossen bleiben.

Die beiden erläuterten Ausführungsbeispiele zur Durchführung des erfindungsgemäßen Verfahrens werden anhand von Zeichnungen noch näher erläutert, es zeigen:
- Figur 1:: Eine schematische Gesamtdarstellung des Meßbereichs mit einem Sternrad,
- Figur 2:: eine Schnittdarstellung des Sternrades,
- Figur 3:: eine schematische Darstellung des Meßbereichs gemäß dem zweiten Ausführungsbeispiel.

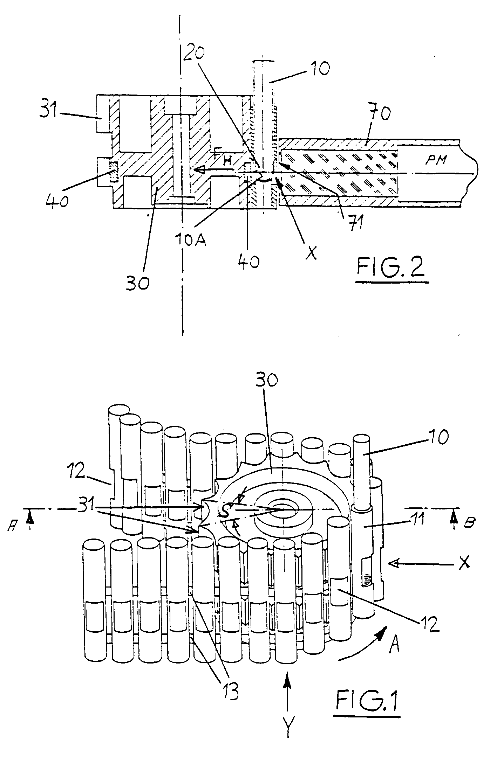

Die Figuren 1 und 2 zeigen die Anwendung des erfindungsgemäßen Verfahrens bei einer Meßanordnung gemäß der DE-OS 36 23 601, bei dem die den Haltern 11 aneinandergeketteten Probengefäße 10 mit Hilfe eines Sternrades 30 an der Meßöffnung 71 (Meßstelle X) eines Fotomultipliers 70 vorbeigeführt werden (Pfeil A in Fig. 1). In der perspektivischen Prinzipdarstellung der Fig. 1 und dem zugehörigen Schnittbild in der Ebene A-B der Fig. 2 befindet sich ein Probenröhrchen 10 gerade vor der Meßöffnung 71 des Fotomultipliers 70. Durch Zugabe der die Chemilumineszenz initiierenden Substanz in das Probenröhrchen 10 werden dann infolge der ablaufenden chemischen Reaktion Photonen freigesetzt, die durch Meßöffnungen 12 in den Haltern 11 durch die Eintrittsöffnung 71 die Verstärkerbauteile des Photomultipliers 70 erreichen.

Die genaue Arbeitsweise und mechanische Anordnung einer solchen Vorrichtung ist in der genannten DE-OS 36 23 601 genauer beschrieben und braucht daher hier nicht weiter erläutert zu werden.

Bei einer solchen Meßvorrichtung besteht die Umsetzung des erfindungsgemäßen Verfahrens nun darin, daß das der Meßöffnung 71 unmittelbar gegenüberliegende Sternrad 30 im Basisbereich jedes Segmentes S (d.h. zwischen zwei aufeinanderfolgenden Lamellen 31) ein Permanentmagnet 40 eingelassen ist. Bei dem dargestellten Ausführungsbeispiel der Fig. 1, wo die Kette der Halter 11 etwa einen Umschlingungswinkel von 180° um das Sternrad 30 bilden, bewirken diese Permanentmagneten 40, daß (gesehen von einem vorbeitransportierten Probengefäß 10 als Koordinatensystem) auf dieses Probengefäß bei entsprechender Polung der Permanentmagneten ein zunächst ansteigendes Magnetfeld einwirkt, das während der Dauer der Umschlingung (also etwa 180° in Fig. 1) konstant bleibt, da dann eine ortsfeste Relation zwischen jedem Probengefäß 10 und "seinem" Permanentmagneten 40 erhalten bleibt.

Die Stärke des Permanentmagneten ist nun so gewählt, daß die von ihm ausgeübte Kraft F_{H} (Fig. 2) ausreicht, um während des Transportes der Probengefäße 10 vom Eintritt der oben erwähnten stationären Bedingungen (Pfeil Y in Fig. 1) bis zur Messung (Pfeil X in Fig.1), also bei einer Bewegung um 90° die in den Probengefäßen 10 in Lösung befindlichen paramagnetischen Mikropartikel 20 an die der Meßöffnung 71 abgewandte Innenwandung 10A des Probengefäßes 10 zu ziehen, so daß diese den optischen Weg der bei der Chemilumineszenz abgegebenen Photonen zur Meßöffnung 71 nicht mehr behindern können. Es soll nochmals hervorgehoben werden, daß die radial nach innen zur Achse des Sternrades 30 gerichtete Kraft F _{H}, bezogen auf den Umschlingungsbereich des Sternrades 30, durch die Probengefäße 10 relativ zu diesen zeitlich und örtlich konstant bleibt, was natürlich nicht ausschließt, daß zur Erzeugung der Kraft ein Gradient des zugehörigen Magnetfeldes gegeben sein muß.

Das Einlassen zylindrisch ausgebildeter Permanentmagnete 40 in entsprechende Bohrungen im Basisbereich der Segmente S in der Höhe der Meßöffnung 71 ist demnach eine besonders einfache und elegante Möglichkeit, das erfindungsgemäße Verfahren bei einer meßanordnung gemäß der DE-OS 36 23 601 umzusetzen.

Fig. 3 zeigt eine ebenso einfache Möglichkeit, den erfindungsgemäßen Effekt zu erzielen bei einem Meßgerät wie in der DE-PS 1 498 857 beschrieben: Hierbei ist eine Gruppe von Probengefäßen 10 einem gemeinsamen Halter 15 zugeordnet, mehrere Halter 15 werden in Pfeilrichtung nach einem "horizontalen Paternoster-Prinzip" an der Meßöffnung 71 des Photomultipliers 70 vorbeibewegt. Hierbei ist dann gegenüber der Meßöffnung 71 und für eine entsprechend lange "Vorlaufstrecke" (entsprechend dem 90°-Bogen beim erstgenannten Ausführungsbeispiel) ein Permanentmagnet 50 angeordnet, dessen Stärke und Länge so gewählt ist, daß er abhängig von der Transportgeschwindigkeit der Halter 15 "rechtzeitig" die Probengefäße 10 derart beeinflußt, daß die dort befindlichen paramagnetischen Mikropartikel an die dem Magneten 50 zugeordnete, von der Meßöffnung 71 abgewandte Innenwandung der Probengefäße 10 gezogen werden und somit den optischen Weg für die bei der Chemilumineszenz freigesetzten Photonen freigeben.

Bei beiden dargestellten Ausführungsbeispielen werden die störenden Magnetpartikel auf der der Meßöffnung 71 gegenüberliegenden Innenwandung der Probengefäße 10 festgehalten. Es ist jedoch grundsätzlich genauso möglich,beispielsweise die Magnete unterhalb der Probengefäße 10 anzuordnen, so daß sich die Magnetpartikel dort absetzen können. Entscheidend ist lediglich, daß diese die Chemilumineszenz-Photonen auf ihrem Weg zum Photomultiplier 70 nicht behindern.

## Patentansprüche

1. Verfahren zur Messung der Chemilumineszenz, bei dem der auszumessende, mit einem luminogenen Marker (label) versehene Komplex (beispielsweise Antigen-Antikörper-Komplex bei einem Immunoassay oder Hybrid bei einem DNS-Sondenassay) zunächst an paramagnetische Mikropartikel angekoppelt worden ist, um mit Hilfe eines Magnetfeldes überschüssige, markierte Substanz abzutrennen, und bei dem die in der Meßlösung dann befindlichen, auszumessenden Antigen-Antikörper-Komplexe bzw. Hybride wieder von den Mikropartikeln getrennt werden und schließlich der Marker (label) aktiviert wird und der entstehende Lichtimpuls in einem Photomultiplier gemessen wird,
dadurch gekennzeichnet, daß für eine bestimmte Zeitdauer vor der Initiierung der Lumineszenzreaktion und Durchführung der Lumineszenzmessung die Probengefäße (10) mit der Meßlösung einem im wesentlichen zeitkonstanten Magnetfeld ausgesetzt werden, dessen resultierende Kraft (F_{H}) auf die Mikropartikel (20) von der Meßöffnung (71) des Photomultipliers (70) weg, insbesondere zur gegenüberliegenden Innenwandung (10A) der Probengefäße (10) gerichtet ist, und die hinsichtlich der Zeitdauer ihrer Einwirkung und ihrer Größe so bemessen ist, daß spätestens während der dann anschließenden Durchführung der Lumineszenzmessung die Mikropartikel (20) sich außerhalb des optischen Weges der Lumineszenz-Photonen zur Meßöffnung (71) des Photomultipliers (70) befinden, insbesondere sich an der der Meßöffnung (71) gegenüberliegenden Innenwandung (10A) ihres jeweiligen Probengefäßes (10) abgesetzt haben.

2. Anordnung zur Durchführung des Verfahrens nach Anspruch 1, bei der die Probengefäße mittels eines gegenüber der Meßstelle angeordneten Sternrades an der Meßstelle vorbeigeführt werden, wo auch die Zugabe der die Chemilumineszenz intitiierenden chemischen Substanz erfolgt, dadurch gekennzeichnet, daß im Basisbereich der die Probengefäße (10) während des Vorbeitransports an der Meßstelle (X) umschließenden Segmente (S) des Sternrades (30) Permanentmagnete (40) angeordnet sind.

3. Anordnung zur Durchführung des Verfahrens nach Anspruch 1, bei der jeweils mehrere Probengefäße in einer linearen Anordnung an der Meßstelle vorbeigeführt werden, wo auch die Zugabe der die Chemilumineszenz intitiierenden chemischen Substanz erfolgt, dadurch gekennzeichnet, daß parallel zur Transportrichtung der Probengefäße (10) und auf ihrer gegenüber der Meßöffnung (71) liegenden Seite ein stabförmiger Permanentmagnet (50) angeordnet ist, der sich zumindest bis zur Meßöffnung (71) erstreckt.

4. Anordnung nach Anspruch 2 oder Anspruch 3, dadurch gekennzeichnet, daß ein magnetisch gegen die Permanentmagnete (40/50) abgeschirmter Photomultiplier (70) verwendet wird.

5. Anordnung nach Anspruch 2 oder Anspruch 3, dadurch gekennzeichnet, daß die Meßöffnung (71) durch den Eingang eines Lichtleiters gebildet wird, dessen Ausgang mit dem Eingang eines räumlich entfernten Photomultipliers gekoppelt ist.

## Claims

1. Method of measuring chemiluminescence, wherein the complex to be measured (for example a complex of antigens and antibodies in an immunoassay or a hybrid in a DNA probe assay), which is provided with a luminous marker (label), has been initially connected to paramagnetic microparticles in order to separate the surplus labelled substance by means of a magnetic field, and wherein the complexes of antigens and antibodies or hybrids, which are then situated in the measuring solution and are to be measured, are separated again from the microparticles, and finally the marker (label) is activated, and the resultant light pulse is measured in a photomultiplier, characterised in that, for a predetermined period of time prior to the luminescence reaction being initiated and the luminescence being measured, the sample containers (10) containing the measuring solution are subjected to a substantially time-constant magnetic field, the resulting force (F_{H}) of said magnetic field upon the microparticles (20) being directed away from the measuring aperture (71) of the photomultiplier (70), more especially being directed towards the oppositely situated internal wall (10A) of the sample containers (10), said force being measured in respect of the duration of its effect and in respect of its size such that, at the latest during the subsequent measuring of the luminescence, the microparticles (20) are situated externally of the optical path of the luminescence photons extending to the measuring aperture (71) of the photomultiplier (70), more especially they have settled on the internal wall (10A) of its respective sample container (10) situated opposite the measuring aperture (71).

2. Arrangement for effecting the method according to claim 1, wherein the sample containers are conducted past the measuring location by means of a star wheel disposed opposite the measuring location, where the chemical substance initiating the chemiluminescence is also added, characterised in that permanent magnets (40) are disposed in the base region of the segments (S) of the star wheel (30) surrounding the sample containers (10) during their conveyance past the measuring location (X).

3. Arrangement for effecting the method according to claim 1, wherein a plurality of sample containers are conducted in a linear disposition past the measuring location, where the chemical substance initiating the chemiluminescence is added, characterised in that a rod-like permanent magnet (50) is disposed parallel to the direction of conveyance of the sample containers (10) and on its side lying opposite the measuring aperture (71) and extends to at least the measuring aperture (71).

4. Arrangement according to claim 2 or claim 3, characterised in that a photomultiplier (70) is used, which is magnetically shielded from the permanent magnets (40/50).

5. Arrangement according to claim 2 or claim 3, characterised in that the measuring aperture (71) is formed by the inlet of a photoconductor, the outlet of which is connected to the inlet of a spaced apart photomultiplier.

## Revendications

1. Méthode de mesure de chimioluminescence, dans laquelle le complexe à mesurer, muni d'un traceur luminogène (label) (complexe antigènes-anticorps, par exemple, pour une immunofluorescence, ou hybride pour une analyse par sonde ADN), est couplé dans un premier temps à des microparticules paramagnétiques, en vue de séparer, à l'aide d'un champ magnétique, la substance excédentaire marquée, et dans laquelle les complexes antigènes-anticorps et/ou les hybrides à mesurer, compris dans la solution de mesure, sont de nouveau séparés des microparticules, le traceur (label) étant enfin activé et l'impulsion lumineuse générée étant mesurée dans un photomutiplicateur, caractérisée en ce que, pour une période définie avant l'initialisation de la réaction de luminescence et la réalisation de la mesure de luminescence, les éprouvettes (10), avec la solution de mesure, sont soumises à un champ magnétique essentiellement constant dans le temps, dont la force résultante (F_{H}) sur les microparticules (20) est dirigée à l'écart de l'orifice de mesure (71) du photomultiplicateur (70), est notamment dirigée en direction de la paroi interne (10A) opposée des éprouvettes (10), cette force étant calculée, pour la durée de son action et sa valeur, de sorte que, pendant la réalisation ultérieure de la mesure de luminescence au plus tard, les microparticules (20) se situent en dehors de la trajectoire optique des photons, produits par la luminescence, en direction de l'orifice de mesure (71) du photomultiplicateur (70), se déposent notamment sur la paroi interne (10A), opposée à l'orifice de mesure (71), de l'éprouvette respective.

2. Montage pour la réalisation de la méthode suivant la revendication 1, dans lequel les éprouvettes sont amenées devant le point de mesure, au moyen d'une roue étoilée disposée en vis-à-vis de ce point, où est également assurée l'addition de la substance chimique, initialisant la chimioluminescence, caractérisé en ce que des aimants permanents (40) sont disposés dans la région de base des segments (S) de la roue étoilée (30), enveloppant les éprouvettes (10), pendant leur transport devant le point de mesure (X).

3. Montage pour la réalisation de la méthode suivant la revendication 1, dans lequel plusieurs éprouvettes sont amenées devant le point de mesure, dans un agencement linéaire, avec une addition parallèle de la substance chimique, initialisant la chimioluminescence, caractérisé en ce qu'un aimant permanent (50) en forme de barre est disposé à la parallèle du sens de transport des éprouvettes (10), sur leur côté opposé à l'orifice de mesure (71), cet aimant s étendant jusqu'à l'orifice de mesure (71), du moins.

4. Montage suivant l'une des revendications 2 et 3, caractérisé par l'utilisation d'un photomultiplicateur (70), protégé de l'effet magnétique des aimants permanents (40/50).

5. Montage suivant l'une des revendications 2 et 3, caractérisé en ce que l'orifice de mesure (71) est formé par l'entrée d'un conduit de lumière, dont la sortie est couplée à l'entrée d'un photomultiplicateur, distant dans l'espace.
